# EUROPEAN PATENT SPECIFICATION

(11) **EP 1 242 587 B1**
(45) Date of publication and mention of the grant of the patent: **25.03.2009**
(21) Application number: 00990883.1
(22) Date of filing: 21.12.2000
(51) Int. Cl.: C12N 9/12, C12Q 1/48

(54) **A NOVEL ISOFORM OF HUMAN cAMP-DEPENDENT PROTEIN KINASE, C ALPHA-s, LOCALIZES TO SPERM MIDPIECE AND THE USE THEREOF**
NEUE ISOFORM DER MENSCHLICHEN CAMP-ABHÄNGIGEN PROTEIN KINASE, C ALPHA-S, MIT LOKALISATION IM SPERMAZELLEN-MITTELTEIL UND DEREN ANWENDUNG
NOUVELLE ISOFORME DE LA PROTEINE KINASE HUMAINE, C ALPHA-s, DEPENDANT DE cAMP, LOCALISEE DANS LA PORTION INTERMEDIAIRE DU SPERME ET SON UTILISATION

(30) Priority: 23.12.1999 NO 996424
(43) Date of publication of application: 25.09.2002
(73) Proprietor: Spermatech AS, 0139 Oslo (NO)
(72) Inventor: Nils Reinton, 0753 Oslo (NO); Sigurd Ørstavik, 0855 Oslo (NO); Tore Jahnsen, 1400 Ski (NO); Bjørn Steen Skålhegg, 1365 Blommenholm (NO); Kjetil Taskén, 0317 Oslo (NO); Trine Haugen, 0687 Oslo (NO)
(74) Representative: Webber, Philip Michael
(86) International application number: PCT/NO2000/000441
(87) International publication number: WO 2001/048170

(56) References cited:
- J.T. SAN AGUSTIN ET AL.: 'The catalytic subunit of sperm cAMP-dependent protein kinase (PKA) is a splice variant of C alpha expressed specifically in the testis in a wide range of mammals' MOLECULAR BIOLOGY OF THE CELL vol. 10, 1999, page 276A, ABSTRACT NO. 1600, XP002940916
- J.T. SAN AGUSTIN ET AL.: 'The catalytic subunit of the cAMP-dependent protein kinase of ovine sperm flagella has a unique amino-terminal sequence' THE JOURNAL OF BIOLOGICAL CHEMISTRY vol. 273, no. 38, 1998, pages 24874 - 24883, XP001009987
- S.J. BEEBE ET AL.: 'Molecular cloning of a tissue-specific protein kinase (C-gamma) from human testis-representing a third isoform for the catalytic subunit cAMP-dependent protein kinase' MOLECULAR ENDOCRINOLOGY vol. 4, no. 3, 1990, pages 465 - 475, XP002940917
- DATABASE GENBANK [Online] F. MALDONADO ET AL.: 'A cDNA clone encoding human cAMP-dependent protein kinase catalytic subunit type alpha', XP002940918 Database accession no. X07767 & NUCLEIC ACIDS RESEARCH vol. 16, no. 16, 1988, pages 8189 - 8190
- D.C. THOMIS ET AL.: 'Mechanism of interferon action' THE JOURNAL OF BIOLOGICAL CHEMISTRY vol. 267, no. 15, 1992, pages 10723 - 10728, XP002940919
- D.E. CUMMINGS ET AL.: 'Cloning of a mouse protein kinase A catalytic subunit pseudogene and chromosomal mapping of C subunit isoforms' MAMMALINA GENOME vol. 5, 1994, pages 701 - 706, XP001012040
- S. WIEMANN ET AL.: 'Rat C-alpha catalytic subunit of the cAMP-dependent protein kinase: cDNA sequence and evidence that it is the only isofmr expressed in myoblasts' BIOCHIMICA ET BIOPHYSICA ACTA vol. 1089, 1991, pages 254 - 256, XP002940921
- DATABASE BIOSIS [Online] DOC. NO. PREV200100126841 J.T. SAN AGUSTIN: 'The unique catalytic subunit of sperm cAMP-dependent protein kinase is the product of an alternative Capha mRNA expressed specifically in spermatogenic cells', XP002941506 Retrieved from STN International Database accession no. 2001:126841 & MOLECULAR BIOLOGY OF THE CELL vol. 11, no. 9, September 2000, pages 3031 - 3044 & DATABASE GENBANK [Online] XP002941221 Database accession no. AF239744

## Description

### FIELD OF THE INVENTION

The present invention relates to a complementary DNA molecule encoding the protein designated Cα-S and a vector comprising said DNA sequence. This invention is also directed to said protein and the use of this protein in diagnosis, treatment and in developing male contraceptives.

### BACKGROUND OF THE INVENTION

Activation of cAMP-dependent protein kinase (PKA) by cAMP elicits initiation and maintenance of flagellar movement in mature spermatozoa [1;2]. The PKA tetrameric holoenzyme, composed of two catalytic subunits (C) and a regulatory subunit (R) dime is activated upon binding of cAMP by release of catalytically active monomeric C subunit [3]. Reactivation of motility of demembranated sperm can be achieved either by cAMP or by active C subunit [4]. Although the underlying mechanisms are still unknown, sperm have been shown to contain a distinct adenylyl cyclase [5;6], PKA type I and II isozymes [7] and phosphodiesterases [8] as well as distinct PKA C isoforms [9-11] implying that sperm have the machinery to generate cAMP and mediate its effects. Furthermore, anchoring of PKA through A-Kinase Anchoring Proteins (AKAPs) to distinct intracellular sites in sperm is believed to be important for regulating sperm motility since disruption of the AKAP-PKA interaction results in motility arrest [12]. Thus, PKA may be essential for sperm function and consequently for fertility.

There is a substantial complexity of PKA isozymes due to heterogeneity in catalytic (Cα, Cβ, Cγ, PrKX [13]) and regulatory (RIα, RIβ, RIIα, RIIβ) subunits. The various R and C subunits are encoded by distinct genes localized on different chromosomes [14]. Several variants of the catalytic subunit in various species have previously been reported. These include human Cα-2 [15], Aplysia Cα-N2 [16], ovine Cα-s [11], bovine Cβ2 [17], mouse Cβ1, Cβ2 and Cβ3 [18], as well as a pseudogene for murine C, Cx [19]. In addition, the PKA subunit isoforms show cell-specific expression and differential regulation in several cells and tissues. They also reveal different subcellular localization, mainly due to binding to AKAPs [20;21].

### SUMMERY OF THE INVENTION

The present invention includes the cloning of a novel human Cα isoform, identified as the human Cα-s protein on the basis of its similarity to a recently characterized Cα isoform which was identified by purification and peptide sequencing of Cα from ovine sperm [11]. The human Cα-s protein was present in testis and ejaculated sperm and Cα-s localized to the midpiece of human sperm. The present invention includes in this respect a cDNA sequence encoding a Cα-s protein and comprises the specific nucleotide sequence shown in SEQ ID NO:1, wherein the said protein is a splice variant of Cα catalytic subunit of cAMP dependent protein kinase.

The present invention is further directed to a vector comprising the said cDNA sequence. The invention also includes a protein characterised by the Cα-s protein specific amino acid sequence shown in SEQ ID NO:2. The invention includes further use of Cα-s protein in preparation of pharmaceuticals, medicaments to manipulate the motility in sperm or for use as contraceptives. Also disclosed are methods and testsytems for screening molecules interacting with Cα-s, and also a kit.

### BRIEF DESCRIPTION OF THE DRAWINGS

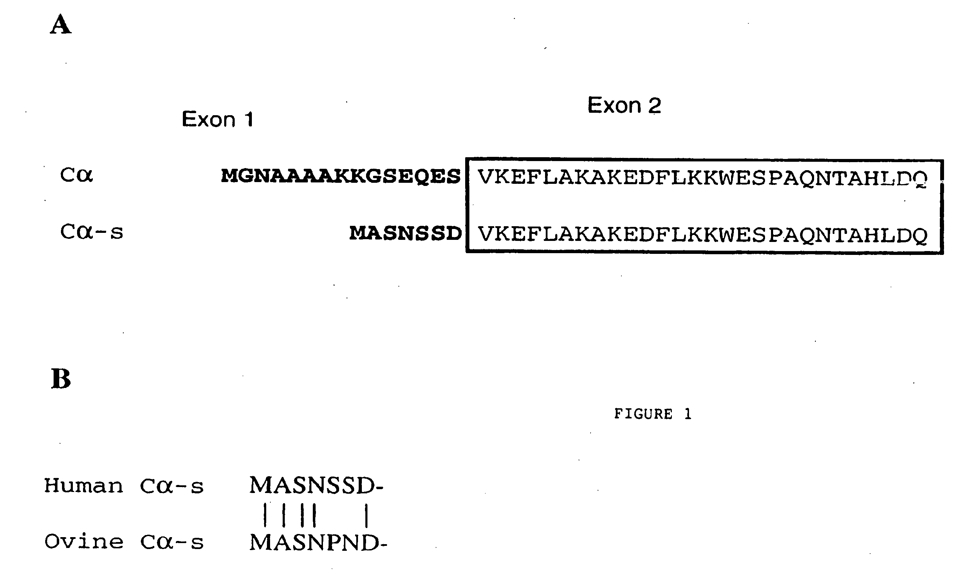

**Fig. 1**. A) Sequence alignment of human Cα isoforms. The cloned Cα-s cDNA sequence was translated into its corresponding amino acid sequence. The peptide sequences of Cα (top) and Cα-s (bottom) that correspond to exons 1 (bold) and the first 28 amino acids of exon 2 (boxed) from the Cα gene are shown B) Sequence comparison of human (top) and ovine (bottom) Cα-s peptide sequences. Vertical lines denote homology.

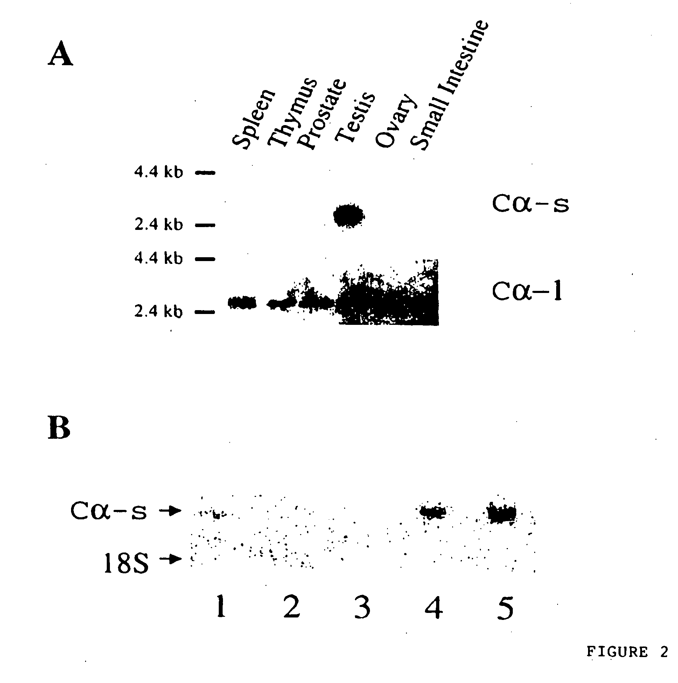

**Fig. 2**. A) Tissue distribution of human Cα mRNAs. A human multiple tissue blot (Clontech Inc., 2µg polyA⁺ RNA per lane) was probed with [³²P]-labeled Cα-s specific (top panel) or a Cα-1 specific (bottom panel) probe (see Materials and Methods). Lines indicate migration of RNA molecular weight markers (kb). B) Germ cell suspensions from human testis were fractionated by centrifugal elutriation, and total RNA was extracted and subjected to Northern blot analysis with a [³²P]-labeled Cα-s specific probe. Fractions were analyzed by flow cytometry for DNA content and separated into haploid cells [round spermatids (RS)] and tetraploid cells [pachytene spermatocytes (PS)]. Diploid cells were considered to be spermatogonia, secondary spermatocytes, or contamination by somatic cells and leucocytes. Lane 1: 57% RS, 7% PS, lane 2: 41% RS, lane 3: 16% RS, 7% PS, lane 4: 21% PS, 3% RS, lane 5: 51% PS, 4% RS. 20 ug of total RNA was loaded with exception of lane 2 (10 ug). The probe detected the Cα-s mRNA of approx 2.8 kb (top arrow) as compared to migration of 18S rRNA (bottom arrow).

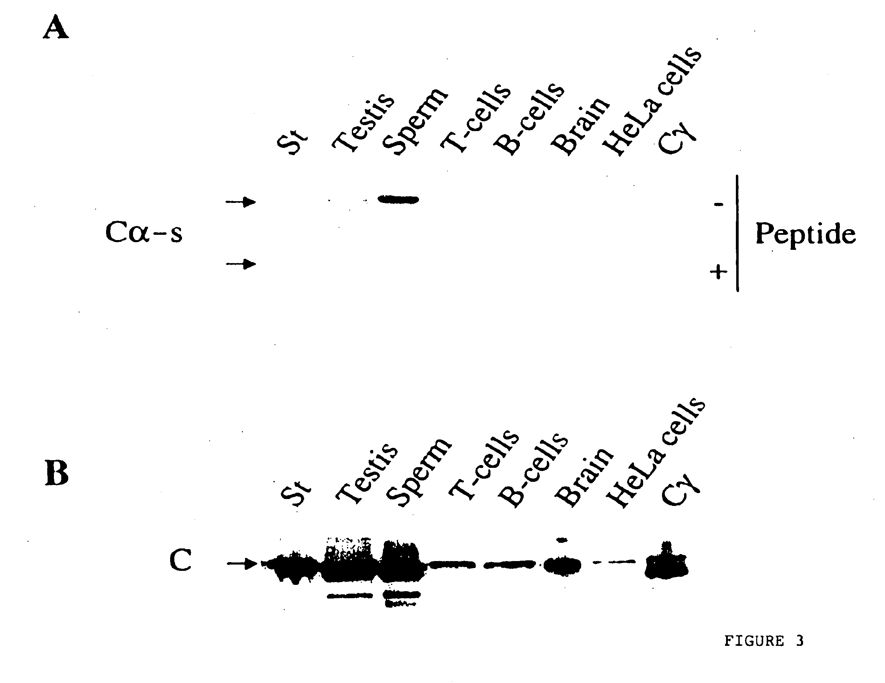

**Fig. 3**. Human Cα-s immunoreactive protein in sperm. Nitrocellulose-immobilized proteins from human cell and tissue lysates were subjected to immunoblot analysis using either the αCα-s antiserum (A) in the absence (upper panel) or presence (lower panel) of 2 mM peptide antigen, or an anti-C antibody reactive to all C-subunit isoforms (B). Lane 1: recombinant murine C standard (500 ng), lane 2: human testis (20 µg protein), lane 3: human sperm lysate (10⁶ sperm), lane 4: human T-cells (20 µg), lane 5: human B-cells (20 µg), lane 6: human brain (20 µg), lane 7: HeLa cells (20 µg), lane 8: recombinant human Cγ (100 ng).

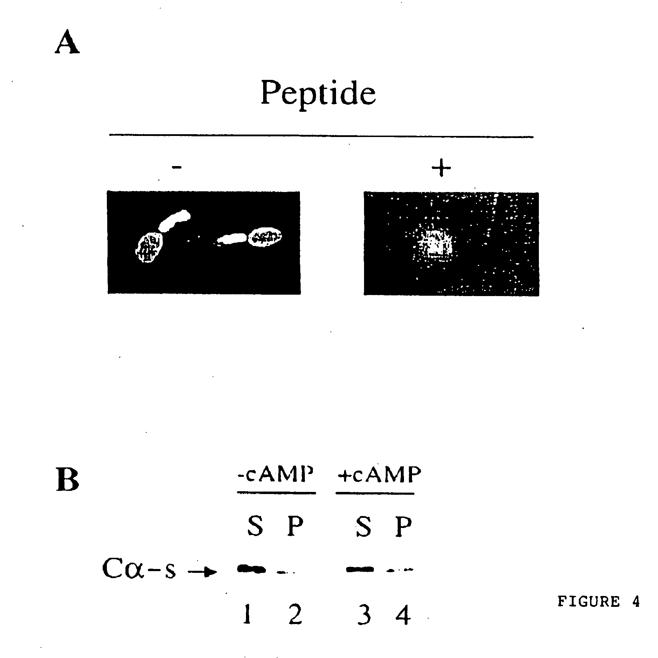

**Fig. 4**. A) Subcellular localization of human Cα-s in sperm cells. Human motile sperm were extracted with 1% Triton X-100, fixed with ethanol on glass slides and subjected to immunofluorescense analysis using the αCα-s antiserum (red staining) in the absence (left panel) or presence of 2 mM peptide antigen (middle panel). Cells were concomitantly DNA-stained with Hoechst (blue). B) Detergent extraction of human sperm tails. Sperm tails were extracted with 1% Triton X-100 (lanes 1 and 2) or 1% Triton X-100 and 20 µM cAMP (lanes 3 and 4), fractionated into soluble (S) and unsoluble (P) fractions and subjected to immunoblotting using the anti Cα-s antibody.

### DETAILED DESCRIPTION OF THE INVENTION

The abbreviations used are: PKA, cAMP-dependent protein kinase; AKAP, A-kinase anchoring protein; C, catalytic -and; R, regulatory subunit of PKA; PBS, phosphate buffered saline.

The present inventors demonstrate the cloning and characterisation of a partial cDNA-sequence encoding Cα-s, an alternatively spliced PKA Cα subunit which contains a novel N-terminus encoded by an alternative exon 1 of the Cα gene. The inventors demonstrate that the Cα-s mRNA is expressed in human male germ cells and that the Cα-s protein is present in ejaculated sperm. Furthermore, the results demonstrate Cα-s localization to detergent soluble and detergent-resistant structures in sperm midpiece. The length of the unique N-terminal 7 amino acid sequence of both ovine and human Cα-s is derived from exon I in the respective species [37]. In addition, the murine Cx pseudogene [19] translates into a predicted N-terminal region (MPSSSND) with the same length that shares homology (4 identical residues out of 6) to both ovine and human Cα-s. Thus, it is possible that the murine Cx pseudogene originated by retroposition of a Cα-s mRNA. This implies the expression of Cα-s in three different species, which would mean that Cα-s has been conserved during evolution. The present invention relates to a cDNA sequence encoding a protein designated Cα-s comprising the specific nucleotide sequence shown in SEQ ID NO: 1 wherein the said protein is a splice variant of a Cα catalytic subunit of cAMP dependent protein kinase. The present invention is further directed to a vector comprising the said cDNA molecule. The invention also includes a protein comprising the protein designated Cα-S specific amino acid sequence shown in SEQ ID NO:2. The invention includes the protein designated Cα-s in preparation of pharmaceuticals, medicaments to manipulate the motility in sperm or for use as contraceptive. Also disclosed are methods and testsytems for screening molecules interacting with Cα-s, a kit.

The inventors have previously described another testis-speclfic C subunit, the Cγ isoform [9], which is present only in primates and is derived from an intronless retroposon [10] Sequence alignment demonstrated that Cα-s is clearly distinct from the Cγ isoform, in agreement with a previous report on ovine Cα-s [11]. However, the expression pattern of Cα-s seen in human male germ cells resembles that of Cγ, and it is possible that these two isoforms utilize similar germ cell-specific promoter elements. Although the exact role of these proteins in human spermatogenesis and sperm function remains to be elucidated, the presence of two distinct C isoforms in the human testis and sperm suggest either a need for substantial redundancy in order to preserve function, or support a notion that different isoforms and alternatively spliced gene products serve distinct and specific sperm functions.

The solubility properties of human Cα-s are reflected by the observation that the presence of both Triton X-100 and cAMP was inadequate to fully extract this protein from sperm midpiece. This is in contrast to the previous report on ovine Cα-s [11] where ovine Cα-s could be extracted only by Triton X-100 when added in combination with cAMP. However, the authors did not account for the insoluble material after this extraction. Thus, it is possible that a pool of Cα-s is insoluble in the presence of cAMP also in ovine sperm. Nevertheless the inventors data support the notion that the Cα-s subunit has unusual solubility properties. An intriguing possibility is that in addition to its anchoring to an R subunit, the shortened Cα-s amino terminus (as compared to the "normal length" Cα), exposes hydrophobic regions that interacts with membraneous or cytoskeletal structures [32]. This would render the free monomeric Cα-s insoluble both in the absence and presence of cAMP. Such a hypothesis is supported by the fact that Cα-s lacks the N-terminal phosphorylation site at Ser10, present in other isoforms of the catalytic subunit [33]. Mutagenesis at Ser10 renders the catalytic subunit insoluble [34] possibly as a result of exposing hydrophobic regions. Independent anchoring of Cα-s may also provide an explanation to the fact that RII knock out mice are fertile [35]. The inventors have recently shown that the RIα and RIIα subunits of PKA are anchored by the A-kinase anchoring protein, AKAP220, to detergent-resistant structures in sperm midpiece whereas S-AKAP84 associate with membrane structures in the same region [36]. Thus, AKAP220/S-AKAP84, RIα/RIIα and Cα-s may constitute signalling complexes present in the midpiece region of human sperm specialised at regulating motility and/or other sperm functions.

In order to determined the functional importance of Cα-s in sperm motility one of the inventors generated knock out mice harbouring a genetic deletion in the gene encoding both variants of Cα, Cα1 and Cα-s (Skålhegg et al, 20000 submitted). Said mice develop morphological normal sperm with a normal appearance. However, the sperm have no motility capacity and PKA phosphotransferase activity. Due to the sperm motility defect, it is assumed that these mice are unfertile. The Cα null mutated mice have in addition 30% growth retardation due to low level of growth hormone. These mice are also sensitive to diseases and only 10 % reach sexual maturity.

Cα-s is an enzyme that is uniquely expressed in the midpiece of elongated mature sperm cells, whereby its function is to regulate sperm motility. Thus, altered levels, location and/or activity of Cα-s will according to our results, modulate the sperm cells ability to swim and fertilize the egg cell. This knowledge can be used to diagnose male infertility, treat cases of male infertility, and to develop male contraceptives.
***1) Sperm cell motility and diagnostics:*** Sperm cell motility-dependent infertility may be caused by mal- or low enzymatic activity by Cα-s, the absence of Cα-s protein or dislocation of Cα-s.
   **1.1)** ***Improving sperm cell motility**:* Present invention makes it possible to identify, characterize and produce pharmacological compositions after high through put screening that specifically will improve the enzymatic activity of Cα**-**s. These compositions should be developed such that they can be introduced directly (by mixing) to the sperm cells in order to improve motility in the context of *in vitro* fertilization or aided fertilization.
   **1.2) *Kits for diagnosing non-motile sperm:*** Sperm cell motility-dependent infertility may be caused by absence of enzymatic active Cα-s. Present invention makes it possible to develop kits, which would diagnostically facilitate if the presence of Cα-s is the cause for non-motile sperm cells. Such kits should be developed with Cα-s specific antibodies, which have already been developed in the inventors laboratory.
**2) *Contraceptives based on C***α***-s activity and location:*** Removing Cα-s activity and/or protein from the sperm midpiece renders the sperm cell non-motile as shown by generating genetic knock out mice lacking the Cαgene.
   **2.1) *Contraceptives based on C***α***-s activity:*** The present invention makes it possible to identify, characterize and produce pharmacological compositions after high through put screening that specifically and irreversibly will inhibit the enzymatic activity of Cα-s in newly synthesised sperm. These compositions should be developed such that they can be introduced orally to enter the blood system reaching the sperm cells.
   **2.2) *Contraceptives based on C***α***-s location:*** The present invention makes it possible to identify, characterize and produce pharmacological compositions after high through put screening that will specifically and irreversibly block Cα-s interaction with the sperm midpiece. These compositions should be developed such that they can be introduced orally to enter the blood system reaching the newly synthesised sperm cells.

The present invention includes a method for inspection and screening of patient spermatozoa for the presence and location of Cα-s protein of the invention comprising:
a) collecting and washing in buffer of ejaculated mature sperm;
b) preparing purified sperm cells for immunofluorescence by letting them settle onto poly L-lysine coated cover slips following detergent-dependent stripping of the sperm cells;
c) incubating with primary antibody (Ab), either irrelevant Ab or Cα-s specific Ab of the invention; washing in washing buffer to remove antibody overshoot and incubating sperm cells with secondary anti-IgG Ab conjugated with a fluorescent and moiety
d) inspecting sperm cells under fluorescent microscopy.

The present invention also includes a method for screening patient spermatozoa for the catalytic activity of the midpiece associated Cα-s protein of the invention comprising:
a) collecting and washing in buffer of ejaculated mature sperm;
b) preparing of purified sperm cells for protein kinase assay by separating head and tail fraction by sonication, centrifugation and lysis of sperm tail in detergent containing buffer;
c) monitoring specific catalytic activity of the protein designated Cα-s herein by established assay. Cα1 activity is used as an internal control to determine relative activity.

### MATERIALS AND METHODS

*Isolation of cDNA Clones by 5'-Rapid Amplification of cDNA Ends (RACE)-*Human testis RACE-Ready cDNA was purchased from Clontech Laboratories Inc. Palo Alto CA (cat. no. 7414-1). A primary PCR with primer Cα-2137 (5'-GGACTTGGCCTCTCCTGTTCCCTTTTG -3', nucleotides 2137 to 2163 in the human Cα cDNA sequence) or primer Cα-1363 (5'-TGTGGGGAAAGAGGAAGGGAAAAGT-3', nucleotides 1363 to 1387 the human Cα cDNA sequence) and anchor specific primer (AP1, Clontech) was performed. A subsequent secondary PCR was performed using primer Cα-188 (5'-AAACTGATCCAAGTGGGCTGTGTTC -3', nucleotides 188 to 212 in the human Cα cDNA sequence) or primer Cα-624 (5'- GCGAAACCGAAGTCTGTCACCTG-3', nucleotides 624 to 646 in the human Cα cDNA sequence), and anchor specific primer (AP2, Clontech). The Clontech Advantage PCR kit (Clontech cat. no. K1905-1) was used in both PCR reactions with 25 cycles of denaturation at 94°C for 30 s and with annealing and extension at 68°C for 4 min. The 848- and 560-base pair PCR products were subcloned and sequenced.

*Sequencing of cDNA Clones*-Plasmids were sequenced on both strands by the dideoxy chain termination method [22] using cycle sequencing protocols (ThermoSequenase kit cat. no. US79760, Amersham Life Science Inc., Cleveland, OH 44128), [³³P]-labeled dideoxynucleotides (cat. no. AH9539, Amersham) and a combination of vector- and insert specific primers. Nucleotide and amino acid sequence data were analyzed using the GCG program package (program manual for the Wisconsin package, version 8, September 1994, Genetics Computer Group, Madison, WI 53711).

*Fractionation of Germ Cells from Human Testis*-Germ cells were isolated by combined treatment with trypsin and DNase [23], followed by separation using a centrifugal elutriation method essentially as described elsewhere [24]. Purity of the cell fractions was evaluated by analysis of DNA content by flow cytometry, and phase contrast microscopy.

*Sperm fractionation*- Mature motile sperm from human donors were purified using the swim-up procedure [25]. Removal of sperm heads was performed by sonicating sperm for 3 min. and subsequent sedimentation of heads at 1500 X g for 5 min. For Triton X-100 extraction, sperm or sperm tails were extracted 30 min with 1% Triton X-100 in the presence or absence of 20 µM cAMP essentially as described elsewhere [11].

*Oligo nucleotide probes*-Specific probes were made using oligo 5'-AAGAAGGGCAGCGAGCAGGAGAGCGTGAAAGAATTCTTAG-3' corresponding to positions 102 to 141 in the human Cα cDNA sequence (Cα-1) and oligo 5'-CTGAGAACAGGACTGAGTGATGGCTTCCAACTCCAGCGAT-3' corresponding to positions 1 to 40 in the human Cα-s cDNA sequence (Cα-s), in an oligomerization protocol as described elsewhere [26].

*Northern Atialysis-*Total RNA from human germ cell fractions was extracted by the guanidine isothiocyanate/CsCl method as previously described [27]. Northern analysis was performed using 10-20 µg RNA/lane. Ethidium bromide staining of the gel verified the loading in each lane. Fiiters with human germ cell fractions or multiple human tissues (Clontech, cat. no. 7759-1) were probed with [³²P]-labeled Cα-) or Cα-s probes.

*Antibodies*-Anti-Cα-s antiserum was made by immunizing rabbits with hemocyanine-coupled synthetic peptides (Antigen EP 990209, NH₂- ASNSSDVKE-CONH₂; Eurosentec, Seraing, Belgium) corresponding to amino acids 1 to 9 of Cα-s and used at a dilution of 1/100 for immunofluorescence. Anti-Cα-s antiserum was enriched for IgG on protein A sepharose columns (Pharmacia, Stockholm, Sweden) and subsequently affinity-purified on columns with peptide coupled to CNBr-activated Sepharose 4B (Pharmacia) and used at 2 µg/ml for immunoblotting. Cγ antibody (Santa Cruz Biotechnology, Inc., Santa Cruz CA, USA, cat. no. sc-905) was used at 2 µg/ml to detect all isoforms of PKA C subunits.

*Immunological procedures*-Immunoblot analysis was performed as described elsewhere [28]. Immunoreactive proteins were detected by horseradish peroxidase-labeled protein A (Amersham) in the second layer and developed using ECL (Amersham cat. no. RPN2106). Immunofluorescence was performed on detergent extracted mature motile sperm from human donors (see above) fixed on poly-L lysine coated glass slides with 3 % paraformaldehyde and permeabilized with 0.5% Triton X-100 for 15 min. Immunofluorescence detection was performed as described previously [29]. DNA was labeled with 0.1 µg/ml Hoechst 33342. Cells were examined on an Olympus AX70 epifluorescence microscope using a 100X objective. Photographs were taken with a Phototonic Science CCD camera and the OpenLab software (Improvision Co., Coventry, UK).

In order that this invention may be better understood, the following examples are set forth. These examples are for the purposes of illustration only, and are not to be construed as limiting the scope of the invention in any manner.

### EXAMPLES

### EXAMPLE 1

*Identification of human C*α*-s.* In order to look for the possible presence of differentially spliced Cα transcripts, 5'-RACE was performed using a human testis cDNA library and primers situated downstream of exon 1. This yielded one clone of 560 bp where the 3' region (nucleotides 40 to 560) shared identity to the previously characterized human Cα-cDNA [30] from nucleotide 126 to 646. Using the BLASTN program [basic local alignment and search tool [31]] similarity to the murine Cx pseudogene [19] was found from position 14 to 560. An ATG start codon was identified (positions 19 to 21), giving rise to 180 amino acids of reading frame that did not contain any stop. Whereas the 173 C-terminal amino acid residues of this partial reading frame was identical to positions 16 to 188 in the human Cα protein, the 7 amino acids (SEQ ID NO: 2, underlined) not present in the previously reported Cα sequence constituted a novel N-terminus. Fig. 1A shows the amino acid sequences corresponding to exon 2 in human Cα(boxed) and the N-terminal amino acids that differ between the two peptide sequences (bold). The N-terminal 7 amino acids were homologous (4 identical amino acids out of 6, in addition to the methionine) to a distinct Cα protein, Cα-s, purified and peptide sequenced from ovine sperm [11](Fig. 1 B), demonstrating that the human Cα splice variant most probably is the Cα-s isoform.

### EXAMPLE 2

*Tissue and Cell Specific Expression of Human C*α*-s mRNA*. To determine which human tissues expressed Cα and Cα-s mRNAs, specific probes for Cα (now designated Cα-1) and Cα-s were radioactively labeled and used to probe a Northern blot with human tissues. As shown in the top panel of Figure 2A, Cα-s mRNA was expressed exclusively in testis. The Cα-1 probe detected expression in all tissues and cell types examined (Fig. 2A, lower panel). To further explore Cα-s mRNA expression in human testis, germ cells were examined for the expression of the Cα-s mRNA (Fig. 2 B). Male human germ cells were isolated and fractionated as described in Materials and Methods. Cα-s mRNA was expressed in fractions 4 and 5, enriched in pachytene spermatocytes (PS) (lanes 4 and 5), but was not detected in fractions 1-3 enriched in round spermatids (RS) (lanes 1-3).

### EXAMPLE 3

*The C*α*-s Protein Is Present in Human Testis and Sperm-An* antiserum directed towards a peptide corresponding to amino acids 1 to 9 of Cα-s was affinity purified and used for immunoblotting analysis. Immunoblotting of human tissue and cell homogenates, revealed a 39-kDa immunoreactive band present in testis and sperm (Fig. 3A, top panel, lanes 2 and 3 respectively), but not in any other cells or tissues examined. (Fig. 3A, top panel, lanes 4 to 7). As expected, heterologously expressed murine Cα and human Cγ proteins did not react with the antiserum (Fig. 3A, lanes 1 and 8 respectively). The immunospecificity of the 39-kDa band in human testis and sperm was demonstrated by competition with the antigen (Fig. 3A, lower panel, lanes 2 and 3 respectively). The presence of other C isoforms in these tissues as well as immunoreactivity of the standards, was demonstrated by probing a parallel blot using an anti-C antibody that recognizes all known C isoforms (Fig. 3 B).

### EXAMPLE 4

*The C*α*-s Protein Localizes to Detergent Resistant Structures in Sperm Midpiece*-The subcellular localization of human Cα-s was examined in sperm by immunofluorescence using the Cα-s antiserum (Fig. 4A, red). In ejaculated sperm preparations extracted with Triton X-100 (Fig 4A, left panel) or left untreated (results not shown), Cα-s labeling was restricted to the midpiece region. The staining was shown to be specific since preimmuneserum or an unrelated antibody did not produce any signal by immunofluorescence in sperm (results not shown), and the staining was competed with the corresponding peptide (Fig. 4A, right panel). To further examine the solubility of Cα-s in human sperm midpiece, we extracted sperm tails with detergent both in the absence and presence of cAMP. Figure 4B shows immunoblotting of insoluble (P) and soluble (S) fractions from human sperm tails extracted with Triton X-100 (lanes 1 and 2) or Triton X-100 in combination with 20 µM cAMP (lanes 3 and 4), using the anti Cα-s antibody. In both cases approximately 2/3 could be solubilized from the particulate fraction. Addition of cAMP to the extraction buffer did not significantly alter the Cα-s signal in either fraction as compared to treatment with Triton X-100 alone, indicating that a pool of Cα-s was insoluble also in the presence of cAMP.

### REFERENCES

[1] Tash JS, Means AR. Regulation of protein phosphorylation and motility of sperm by cyclic adenosine monophosphate and calcium. Biol. Reprod. 1982; 26: 745-763.
[2] Brokaw CJ. A lithium-sensitive regulator of sperm flagellar oscillation is activated by cAMP-dependent phosphorylation. J. Cell Biol. 1987; 105: 1789-1798.
[3] Beebe SJ, Corbin JD. Cyclic nucleotide-dependent protein kinases. The Enzymes 1986; 17: 43-111.
[4] San Agustin JT, Witman GB. Role of cAMP in the reactivation of demembranated ram spermatozoa. Cell Motil. Cytoskeleton 1994; 27: 206-218.
[5] Gordeladze JO, Hansson V. Purification and kinetic properties of the soluble Mn2+-dependent adenylyl cyclase of the rat testis. Mol. Cell Endocrinol. 1981; 23: 125-136.
[6] Buck J, Sinclair ML, Schapal L, Cann MJ, Levin LR. Cytosolic adenylyl cyclase defines a unique signaling molecule in mammals. Proc. Natl. Acad. Sci. U. S. A 1999; 96: 79-84.
[7] Landmark BF, Oyen O Skalhegg BS, Fauske B, Jahnsen T, Hansson V. Cellular location and age-dependent changes of the regulatory subunits of cAMP-dependent protein kinase in rat testis. J. Reprod. Fertil. 1993; 99: 323-334.
[8] Salanova M, Chun SY, Iona S, Puri C, Stefanini M, Conti M. Type 4 cyclic adenosine monophosphate-specific phosphodiesterases are expressed in discrete subcellular compartments during rat spermiogenesis. Endo 1999; 140: 2297-2306.
[9] Beebe SJ, Øyen O Sandberg M, Frøysa A, Hansson V, Jahnsen T. Molecular cloning of a tissue-specific protein kinase (C gamma) from human testis-representing a third isoform for the catalytic subunit of cAMP-dependent protein kinase. Mol. Endocrinol. 1990; 4: 465-75.
[10] Reinton N, Haugen TB, Orstavik S, Skalhegg BS, Hansson V, Jahnsen T, Tasken K. The gene encoding the C gamma catalytic subunit of cAMP-dependent protein kinase is a transcribed retroposon. Genomics 1998; 49: 290-297.
[11] San Agustin JT, Leszyk JD, Nuwaysir LM, Witman GB. The catalytic subunit of the cAMP-dependent protein kinase of ovine sperm flagella has a unique amino-terminal sequence. J. Biol. Chem. 1998; 273: 24874-24883.
[12] Vijayaraghavan S, Goueli SA, Davey MP, Carr DW Protein kinase A-anchoring inhibitor peptides arrest mammalian sperm motility. J. Biol. Chem. 1997; 272: 4747-4752.
[13] Zimmermann B, Chiorini JA, Ma Y, Kotin RM, Herberg FW. PrKX is a novel catalytic subunit of the cAMP-dependent protein kinase regulated by the regulatory subunit type I. J. Biol. Chem. 1999; 274: 5370-5378.
[14] Tasken K, Naylor SL, Solberg R, Jahnsen T. Mapping of the gene encoding the regulatory subunit RII alpha of cAMP- dependent protein kinase (locus PRKAR2A) to human chromosome region 3p21.3-p21.2. Genomics 1998; 50: 378-381.
[15] Thomis DC, Floyd-Smith G, Samuel CE. Mechanism of interferon action. cDNA structure and regulation of a novel splice-site variant of the catalytic subunit of human protein kinase A from interferon-treated human cells. J. Biol. Chem. 1992; 267: 10723-10728.
[16] Beushausen S, Lee E, Walker B, Bayley H. Catalytic subunits of Aplysia neuronal cAMP-dependent protein kinase with two different N termini. Proc. Natl. Acad. Sci. U. S. A 1992; 89: 1641-1645.
[17] Wiemann S, Kinzel V, Pyerin W. Isoform Cβ2, an unusual form of the bovine catalytic subunit of cAMP-dependent protein kinase. J. Biol. Chem. 1991; 266: 5140-5146
[18] Guthrie CR, Skalhegg BS, McKnight GS. Two novel brain-specific splice variants of the murine Cbeta gene of cAMP-dependent protein kinase. J. Biol. Chem. 1997; 272: 29560-29565.
[19] Cummings DE, Edelhoff S, Disteche CM, McKnight GS. Cloning of a mouse protein kinase A catalytic subunit pseudogene and chromosomal mapping of C subunit isoforms. Mamm. Genome 1994; 5: 701-706.
[20] Scott JD. Cyclic nucleotide-dependent protein kinases. Pharmacol. Ther. 1991; 50: 123-145.
[21] Rubin CS. A kinase anchor proteins and the intracellular targeting of signals carried by cyclic AMP. Biochim. Biophys. Acta 1994; 1224: 467-479.
[22] Sanger F, Nicklen S, Coulson AR. DNA sequencing with chain-terminating inhibitors. Proc. Natl. Acad. Sci. U. S. A. 1977; 74: 5463-5467.
[23] Grootegoed JA, Peters MJ, Mulder E, Rommerts FF, van der Molen HJ. Absence of a nuclear androgen receptor in isolated germinal cells of rat testis. Mol. Cell Endocrinol. 1977; 9: 159-167.
[24] Blanchard Y, Lavault MT, Quernee D, Le Lannou D, Lobel B, Lescoat D. Preparation of spermatogenic cell populations at specific stages of differentiation in the human. Mol. Reprod. Dev. 1991; 30: 275-282.
[25] Wikland M, Wik O Steen Y, Qvist K, Soderlund B, Janson PO. A self-migration method for preparation of sperm for in-vitro fertilization. Hum. Reprod. 1987; 2: 191-195.
[26] Paquette J, Sapienza C. A reliable method for the use of oligonucleotides as probes in blot- hybridization experiments. Mamm. Genome 1992; 3: 1-4.
[27] Knutsen HK, Reinton N, Tasken KA, Hansson V, Eskild W. Regulation of protein kinase A subunits by cyclic adenosine 3',5'- monophosphate in a mouse Sertoli cell line (MSC-1): induction of RII beta messenger ribonucleic acid is independent of continuous protein synthesis. Biol. Reprod. 1996; 55: 5-10.
[28] Witczak O Skalhegg BS, Keryer G, Bornens M, Tasken K, Jahnsen T, Orstavik S. Cloning and characterization of a cDNA encoding an A-kinase anchoring protein located in the centrosome, AKAP450. EMBO J. 1999; 18: 1858-1868.
[29] Collas P, Courvalin JC, Poccia D. Targeting of membranes to sea urchin sperm chromatin is mediated by a lamin B receptor-like integral membrane protein. J. Cell Biol. 1996; 135: 1715-1725.
[30] Maldonado F, Hanks SK. A cDNA clone encoding human cAMP-dependent protein kinase catalytic subunit Cα. Nucl. Acids Res. 1988; 16: 8189-8190.
[31] Altschul SF, Gish W, Miller W, Myers EW, Lipman DJ. Basic local alignment search tool. J. Mol. Biol. 1990; 215: 403-410.
[32] Herberg FW, Zimmermann B, McGlone M, Taylor SS. Importance of the A-helix of the catalytic subunit of cAMP-dependent protein kinase for stability and for orienting subdomains at the cleft interface. Protein Sci. 1997; 6: 569-579.
[33] Yonemoto W, Garrod SM, Bell SM, Taylor SS. Identification of phosphorylation sites in the recombinant catalytic subunit of cAMP-dependent protein kinase. J. Biol. Chem. 1993; 268: 18626-18632.
[34] Yonemoto W, McGlone ML, Grant B, Taylor SS. Autophosphorylation of the catalytic subunit of cAMP-dependent protein kinase in Escherichia coli. Protein Eng 1997; 10:915-925.
[35] Burton KA, Treash-Osio B, Muller CH, Dunphy EL, McKnight GS. Deletion of Type IIalpha Regulatory Subunit Delocalizes Protein Kinase A in Mouse Sperm without Affecting Motility or Fertilization. J. Biol. Chem. 1999, 274: 24131-24136.
[36] Reinton, N., P. Collas, T. Haugen, B.S. Skålhegg, V. Hansson, T. Jahnsen and., Taskén, K. A human A-kinase anchoring protein, hAKAP220, localizes to male germ cell centrosomes and sperm midpiece. Dev Biol 2000; 223, 194-204
[37] Desseyn,J.L., Burton,K.A., & McKnight,G.S. (2000) Expression of a nonmyristylated variant of the catalytic subunit of protein kinase A during male germ-cell development. Proc. Natl. Acad. Sci. U.S. A, 97, 6433-6438.

### SEQUENCE LISTING

SEQ ID NO: 1
   <110> Nils Reinton, Sigurd Ørstavik, Trine B. Haugen, Tore Jahnsen, Kjetil Taskén og Bjørn S. Skålhegg
   <120> **A NOVEL ISOFORM OF HUMAN cAMP-DEPENDENT PROTEIN KINASE,**
      **C**α**-s, LOCALIZES TO SPERM MIDPIECE AND THE USE THEREOF**
   <160> 2
   <210> 1
      <211> 560 nucleotides
      <212> cDNA
      <213> Homo sapiens
      **C**α**-s cDNA sequence**
   <400> 1 
1   tgagaacagg actgagtgat ggcttccaac tccagcgatg tgaaagaatt
cttagccaaa
61  gccaaagaag attttcttaa aaaatgggaa agtcccgctc agaacacagc
ccacttggat
121 cagtttgaac gaatcaagac cctcggcacg ggctccttcg ggcgggtgat
gctggtgaaa
181 cacaaggaga ccgggaacca ctatgccatg aagatcctcg acaaacagaa
ggtggtgaaa
241 ctgaaacaga tcgaacacac cctgaatgaa aagcgcatcc tgcaagctgt
caactttccg
301 ttcctcgtca aactcgagtt ctccttcaag gacaactcaa acttatacat
ggtcatggag
361 tacgtgcccg gcggggagat gttctcacac ctacggcgga tcggaaggtt
cagtgagccc
421 catgcccgtt tctacgcggc ccagatcgtc ctgacctttg agtatctgca
ctcgctggat
481 ctcatctaca gggacctgaa gccggagaat ctgctcattg accagcaggg
ctacattcag
541 gtgacagact tcggtttcgc
SEQ ID NO: 2
   <110> Nils Reinton, Sigurs Ørstavik, Trine B. Haugen, Tore Jahnsen, Kjetil Taskén og Bjørn S. Skålhegg
   <120> **A NOVEL ISOFORM OF HUMAN cAMP-DEPENDENT PROTEIN KINASE,C**α**-s,**
      **LOCALIZES TO SPERM MIDPIECE AND THE USE THEREOF**
   <160> 2
   <210> 2
      <211> 180 aa
      <212> aminoacids
      <213> Homo sapiens
      **C**α**-s Protein sequence**
   <400> 1

Met-Ala-Ser-Asn-Ser-Ser-Asp-Val-Lys-Glu-PheLeu-Ala-Lys-Ala-
Lys-Glu-Asp-Phe-Leu-Lys-Lys-Trp-Glu-Ser-Pro-Ala-Gln-Asn-
Thr-Ala-His-Leu-Asp-Gln-Phe-Glu-Arg-Ile-Lys-Thr-Leu-Gly-
Thr-Gly-Ser-Phe-Gly-Arg-Val-Met-Leu-Val-Lys-His-Lys-Glu-
Thr-Gly-Asn-His-Tyr-Ala-Met-Lys-Ile-Leu-Asp-Lys-Gln-Lys-
Val-Val-Lys-Leu-Lys-Gln-Ile-Glu-His-Thr-Leu-Asn-Glu-Lys-
Arg-Ile-Leu-Gln-Ala-Val-Asn-Phe-Pro-Phe-Leu-Val-Lys-Leu-
Glu-Phe-Ser-Phe-Lys-Asp-Asn-Ser-Asn-Leu-Tyr-Met-Val-Met-
Glu-Tyr-Val-Pro-Gly-Gly-Glu-Met-Phe-Ser-His-Leu-Arg-Arg-
Ile-Gly-Arg-Phe-Ser-Glu-Pro-His-Ala-Arg-Phe-Tyr-Ala-Ala-
Gln-Ile-Val-Leu-Thr-Phe-Glu-Tyr-Leu-His-Ser-Leu-Asp-Leu-
Ile-Tyr-Arg-Asp-Leu-Lys-Pro-Glu-Asn-Leu-Leu-Ile-Asp-Gln-
Gln-Gly-Tyr-Ile-Gln-Val-Thr-Asp-Phe-Gly-Phe-Ala

## Claims

1. A cDNA molecule comprising the nucleotide sequence of SEQ ID NO: 1 encoding a protein designated Cα-s, wherein said protein is a splice variant of a Cα catalytic subunit of cAMP dependent protein kinases.

2. A cDNA molecule encoding the protein having the amino acid sequence of SEQ ID NO: 2.

3. A vector comprising the cDNA molecule according to claim 1 or claim 2.

4. The protein having the amino acid sequence of SEQ ID NO: 2.

5. A protein encoded by the cDNA molecule of claim 1.

6. A protein encoded by the cDNA molecule of claim 1 comprising the specific protein designated Cα-s amino acid sequence of SEQ ID NO: 2.

7. Antibodies to the peptide consisting of amino acids 1-9 of the protein designated Cα-s according to SEQ ID NO: 2.

8. The protein designated Cα-s of any one of claims 4-6, for use as a pharmaceutical.

9. The protein designated Cα-s of any one of claims 4-6, for manipulating the motility in sperm.

10. The use of the protein designated Cα-s of any one of claims 4-6, for the preparation of a medicament for use as a contraceptive.

11. A DNA molecule that is complementary to the entire length of the cDNA molecule of claim 1.

12. The use of a DNA molecule which is complementary to the entire length of the Cα-s cDNA molecule according to claim 1 for the preparation of an anti sense drug.

13. A method for inspection and screening of patient spermatozoa for the presence and location of protein designated Cα-s of claims 4-6, comprising:
a) collecting and washing in buffer of ejaculated mature sperm;
b) preparing purified sperm cells for immunofluorescence by letting them settle onto poly L-lysine coated cover slips following detergent-dependent stripping of the sperm cells;
c) incubating with primary antibody, either irrelevant antibody or Cα-s specific antibody as claimed in claim 7, washing with washing buffer to remove antibody overshoot, and incubating sperm cells with secondary anti-IgG antibody conjugated with a fluorescent moiety; and
d) inspecting sperm cells under fluorescent microscopy.

14. Method of screening patient spermatozoa for the catalytic activity of the midpiece associated protein designated Cα-s of claims 4-6, comprising:
a) collecting and washing in buffer of ejaculated mature sperm;
b) preparing of purified sperm cells for protein kinase assay by separating head and tail fraction by sonication, centrifugation and lysis of sperm tail in detergent containing buffer; and
c) monitoring specific catalytic activity of protein designated Cα-s by established assay, Cα1 activity is used as an internal control to determine relative activity.

## Patentansprüche

1. cDNA-Molekül, das die Nukleotidsequenz der SEQ ID Nr. 1 aufweist, die bzw. das für ein mit Cα-s bezeichnetes Protein kodiert, wobei das Protein eine Splicevariante einer katalytischen Untereinheit Cα cAMP-abhängiger Proteinkinasen darstellt.

2. cDNA-Molekül, das für das Protein mit der Aminosäuresequenz der SEQ ID Nr. 2 kodiert.

3. Vektor, der das cDNA-Molekül nach Anspruch 1 oder 2 enthält.

4. Protein mit der Aminosäuresequenz der SEQ ID Nr. 2.

5. Protein, das durch das cDNA-Molekül nach Anspruch 1 kodiert wird.

6. Protein, das durch das cDNA-Molekül nach Anspruch 1 kodiert wird, und die spezifische Aminosäuresequenz des als Cα-s bezeichneten Proteins der SEQ ID Nr. 2 enthält.

7. Antikörper zum Peptid, das aus den Aminosäuren 1 bis 9 des als Cα-s bezeichneten Proteins gemäß SEQ ID Nr. 2 besteht.

8. Als Cα-s bezeichnetes Protein nach einem der Ansprüche 4 bis 6 zur Verwendung als Medikament.

9. Als Cα-s bezeichnetes Protein nach einem der Ansprüche 4 bis 6 zur Manipulation der Spermienmotilität.

10. Verwendung des als Cα-s bezeichneten Proteins nach einem der Ansprüche 4 bis 6 zur Herstellung eines Medikaments zur Verwendung als Kontrazeptivum.

11. DNA-Molekül, welches zu der gesamten Länge des cDNA-Moleküls des Anspruchs 1 komplementär ist.

12. Verwendung eines DNA-Moleküls, welches zu der gesamten Länge des Cα-s-cDNA-Moleküls nach Anspruch 1 komplementär ist, zur Herstellung eines Antisense-Medikaments.

13. Verfahren zur Untersuchung und zum Screening der Spermatozoen eines Patienten auf die Anwesenheit bzw. das Vorliegen und die Lokalisierung des mit Cα-s bezeichneten Proteins der Ansprüche 4 bis 6 hin, das umfasst:
a) Sammeln und Waschen ejakulierten reifen Spermas in Puffer;
b) Herstellen von gereinigten Spermazellen für die Immunfluoreszenz durch Absinkenlassen bzw. Ablagern derselben auf Poly-L-Lysinbeschichteten Deckgläsern, gefolgt von tensidabhängigem Strippen bzw. Austreiben der Spermienzellen;
c) Inkubieren mit einem Primärantikörper, entweder einem irrelevanten bzw. unspezifischen Antikörper, oder einem spezifischen Cα-s-Antikörper nach Anspruch 7, Waschen mit Waschpuffer zum Entfernen des Antikörperüberschusses, und Inkubieren der Spermienzellen mit einem sekundären Anti-IgG-Antikörper, der mit einem fluoreszierenden Rest bzw. einer fluoreszierenden Einheit konjugiert ist; und
d) Untersuchen der Spermienzellen unter bzw. durch Fluoreszenzmikroskopie.

14. Verfahren zum Screenen der Spermatozoen eines Patienten auf die katalytische Aktivität des mittelstück- bzw. halsassoziierten, mit Cα-s bezeichneten Proteins der Ansprüche 4 bis 6 hin, das umfasst:
a) Sammeln und Waschen ejakulierten, reifen Spermas in Puffer;
b) Herstellen von gereinigten Spermienzellen für den Proteinkinase-Assay durch Trennen der Kopf- und Schwanzfraktion durch Ultraschallbehandlung, Zentrifugation und Lyse des Spermienschwanzes in tensidhaltigem Puffer; und
c) Überwachen der spezifischen katalytischen Aktivität des mit Cα-s bezeichneten Proteins durch einen etablierten Assay, wobei die Cα1-Aktivität als eine interne Kontrolle zur Bestimmung der relativen Aktivität verwendet wird.

## Revendications

1. Molécule d'ADNc comportant la séquence nucléotidique de la SEQ ID N° : 1 codant pour une protéine désignée Cα-s, dans laquelle ladite protéine est un variante d'épissage d'une sous-unité catalytique Cα des protéines kinases cAMP-dépendantes.

2. Molécule d'ADNc codant pour la protéine ayant la séquence d'acides aminés de la SEQ ID N° : 2.

3. Vecteur comportant la molécule d'ADNc selon la revendication 1 ou la revendication 2.

4. Protéine ayant la séquence d'acides aminés de la SEQ ID N° : 2.

5. Protéine codée par la molécule d'ADNc de la revendication 1.

6. Protéine codée par la molécule d'ADNc de la revendication 1, comportant la séquence d'acides aminés de la protéine spécifique désignée Cα-s de la SEQ ID NI : 2.

7. Anticorps dirigés contre le peptide constitué des acides aminés 1 à 9 de la protéine désignée Cα-s selon la SEQ ID N° : 2.

8. Protéine désignée Cα-s selon l'une quelconque des revendications 4 à 6 pour une utilisation en tant que substance pharmaceutique.

9. Protéine désignée Cα-s selon l'une quelconque des revendications 4 à 6, pour manipuler la motilité dans le sperme.

10. Utilisation de la protéine désignée Cα-s selon l'une quelconque des revendications 4 à 6, pour la préparation d'un médicament destiné à une utilisation en tant que contraceptif.

11. Molécule d'ADN qui est complémentaire de la longueur complète de la molécule d'ADNc de la revendication 1.

12. Utilisation d'une molécule d'ADN qui est complémentaire de la longueur complète de la molécule d'ADNc de Cα-s selon la revendication 1 pour la préparation d'un médicament antisens.

13. Procédé d'examen et de criblage des spermatozoïdes d'un patient afin de déterminer la présence et l'emplacement de la protéine désignée Cα-s des revendications 4 à 6, comportant les étapes consistant à :
a) recueillir et laver dans du tampon du sperme mature éjaculé ;
b) préparer des cellules spermatiques purifiées en vue d'une immunofluorescence en les laissant se fixer sur des lamelles recouvertes de poly L-lysine après perméabilisation des cellules spermatiques à l'aide d'un détergent ;
c) incuber à l'aide d'un anticorps primaire, soit un anticorps non spécifique soit un anticorps spécifique à Cα-s tel que revendiqué dans la revendication 7, laver avec un tampon de lavage afin d'éliminer l'excès d'anticorps, et incuber les cellules spermatiques avec un anticorps secondaire IgG conjugué à un groupement fonctionnel fluorescent ; et
d) examiner les cellules spermatiques par microscopie fluorescente.

14. Procédé de criblage des spermatozoïdes d'un patient afin de déterminer l'activité catalytique de la protéine associée à la pièce intermédiaire désignée Cα-s des revendications 4 à 6, comportant les étapes consistant à :
a) recueillir et laver dans du tampon du sperme mature éjaculé ;
b) préparer des cellules spermatiques purifiées en vue d'une analyse de la protéine kinase en séparant les fractions de tête et de queue par sonication, centrifugation et lyse de la queue du sperme dans un tampon contenant un détergent ; et
c) surveiller l'activité catalytique spécifique de la protéine désignée Cα-s par l'intermédiaire de l'analyse établie, l'activité de Cal servant de contrôle interne afin de déterminer l'activité relative.
